# EUROPÄISCHE PATENTANMELDUNG

(11) **EP 0 916 724 A1**
(43) Veröffentlichungstag der Anmeldung: **19.05.1999**
(21) Anmeldenummer: 98119901.1
(22) Anmeldetag: 21.10.1998
(51) Int. Cl.: C12M 1/33, C12M 1/04

(54) **Verfahren und Vorrichtung zur Herstellung von Enzymen**

(30) Priorität: 11.11.1997 DE 19749735
(71) Anmelder: Invent GmbH - Entwicklung Neuer Technologien, 91080 Uttenreut (DE); Technische Consult Tecon GmbH, 38678 Clausthal-Zellerfeld (DE); APPLIKATIONS- UND TECHNIKZENTRUM FÜR ENERGIEVERFAHRENS-, UMWELT-, UND STRÖMUNGSTECHNIK, 92237 Sulzbach-Rosenberg (DE)
(72) Erfinder: Genenger, Bernd, 91077 Hetzles (DE); Vogelpohl, Alfons, 38678 Clausthal-Zellerfeld (DE); Bischof, Franz, 92237 Sulzbach-Rosenberg (DE)
(74) Vertreter: Gassner, Wolfgang, Dr.-Ing.

(57) **Zusammenfassung**

Die Erfindung betrifft ein Verfahren zur Herstellung von Enzymen aus in einer Flüssigkeit vorliegenden Mikroorganismen mit folgenden Schritten:
a) Umwälzung der Flüssigkeit durch zwei gegeneinander gerichtete eine erste Strömungsgeschwindigkeit aufweisende Triebsstrahlen T1, T2 und
b) Aufschluß der Mikroorganismen durch Erzeugen einer zweiten Strömungsgeschwindigkeit, die höher als die erste Strömungsgeschwindigkeit ist.

## Beschreibung

Die Erfindung betrifft ein Verfahren zur Herstellung von Enzymen. Sie betrifft außerdem eine Vorrichtung zur Herstellung von Enzymen nach dem Oberbegriff des Anspruchs 10.

Zur Herstellung von Enzymen werden aerobe Mikroorganismen fermentiert und anschließend aufgeschlossen. Die Fermentation der in einer Flüssigkeit vorliegenden Mikroorganismen erfolgt üblicherweise in einem Rührkesselreaktor, welcher mit Luft begast wird. Anschließend wird die Flüssigkeit aus dem Rührkesselreaktor entnommen, die Mikroorganismen werden aus der Flüssigkeit abgetrennt und in einer separaten Vorrichtung mechanisch aufgeschlossen. Es ist bekannt, die zum Zellaufschluß erforderlichen Scherkräfte durch zwei aufeinandergerichtete Flüssigkeitsstrahlen zu erzeugen (Bomberg, A.; Krämer, P., Cell Disintigration by highly Turbulent Luiquid Impingement Jets, Dechema Biotechnology Conferences Vol. 2, Frankfurt 1988).

Das bekannte Verfahren ist in mehrfacher Hinsicht nachteilig:
a) in den bekannten Rührkesselreaktoren können wegen des relativ geringen volumenbezogenen Stoffübergangskoeffizienten für den Sauerstofftransport aus der gasförmigen in die flüssige Phase nur relativ geringe Biomassegehalte erzeugt werden und
b) der apparative Aufwand zur Durchführung des Verfahrens ist hoch, weil zur Fermentation, Abtrennung und zum Aufschluß jeweils besondere Vorrichtungen in Hintereinanderschaltung erforderlich sind.

Aus der DE 44 18 287 C2 ist eine Vorrichtung zum Mischen zweier Fluide bekannt. Dabei werden zwei parallel zur Behälterachse gerichtete Triebstrahlen in Leitrohren erzeugt. Die Triebstrahlen werden mittels der Leitrohre um 90° umgelenkt und gegeneinander gerichtet. Die bekannte Vorrichtung eignet sich nicht zum Aufschluß von Mikroorganismen. Durch die Triebstrahlen kann hier die erforderliche Scherkraft nicht aufgebracht werden.

Aufgabe der vorliegenden Erfindung ist es, die Nachteile nach dem Stand der Technik zu beseitigen. Es soll insbesondere ein Verfahren zur Herstellung von Enzymen angegeben werden, das ohne großen apparativen Aufwand durchführbar ist. Ferner soll eine Vorrichtung bereitgestellt werden, mit der eine effiziente Herstellung von Enzymen ermöglicht wird.

Diese Aufgabe wird durch die Merkmale der Ansprüche 1 und 10 gelöst. Zweckmäßige Ausgestaltungen der Erfindung ergeben sich aus den Merkmalen der Ansprüche 2 bis 9 und 11 bis 18.

Zur Lösung der Aufgabe ist ein Verfahren zur Herstellung von Enzymen aus in einer Flüssigkeit vorliegenden Mikroorganismen mit folgenden Schritten vorgesehen:
a) Umwälzung der Flüssigkeit durch zwei gegeneinander gerichtete eine erste Strömungsgeschwindigkeit aufweisende Triebstrahle und
b) Aufschluß der Mikroorganismen durch Erzeugen einer zweiten Strömungsgeschwindigkeit, die höher als die erste Strömungsgeschwindigkeit ist.

Das Verfahren kann in einer einzigen Vorrichtung durchgeführt werden. Die zur Durchführung des Verfahrens erforderliche vorbereitende Sterilisation bleibt auf die einzige Vorrichtung beschränkt. Es können hohe Biomassekonzentrationen in der Flüssigkeit realisiert werden.

Vorteilhafterweise beträgt die erste Strömungsgeschwindigkeit höchstens 30 m/s, vorzugsweise 15 m/s. Dadurch wird ein guter Kontakt der Gasphase mit der Flüssigkeit gewährleistet. Die zweite Strömungsgeschwindigkeit beträgt zweckmäßigerweise zwischen 30 m/s und 100 m/s, vorzugsweise 80 m/s. Die zweite Strömungsgeschwindigkeit gewährleistet einen vollständigen Aufschluß der Mikroorganismen.

Nach einem weiteren Ausgestaltungsmerkmal wird der Flüssigkeit während des Schritts lit. a eine Nährstofflösung für die Mikroorganismen und/oder Gas zugeführt. Gleichzeitig kann Flüssigkeit entnommen werden. Aus der entnommenen Flüssigkeit werden die Mikroorganismen vorteilhafterweise abgetrennt und zurückgeführt. Der Aufschluß, d.h. der Schritt lit. b, wird zweckmäßigerweise ohne Zufuhr Nährstofflösung und/oder Gas durchgeführt.

Nach der vorrichtungseitigen Maßgabe der Erfindung ist vorgesehen, daß die Mittel zur Erzeugung der Triebstrahlen eine gemeinsame im wesentlichen senkrecht zur Behälterachse stehende Achse aufweisen, so daß die daraus austretenden Triebstrahlen einander entgegengesetzt sind. Eine solche Anordnung ermöglicht die Fermentation und den Aufschluß in einer einzigen Vorrichtung.

Nach einem Ausgestaltungsmerkmal sind die Zuläufe durch ein in das Behalterinnere ragendes Rohrstück gebildet. Vorteilhafterweise weist das Mittel zur Erzeugung des Triebstrahls mindestens eine Düse, vorzugsweise eine 2-Stoffdüse, auf. In mindestens zwei einander gegenüberliegenden Zuläufen kann die jeweils mindestens eine Düse des Mittels zur Erzeugung eines Triebstrahls aufgenommen sein. So werden besonders hohe Scherkräfte erzielt.

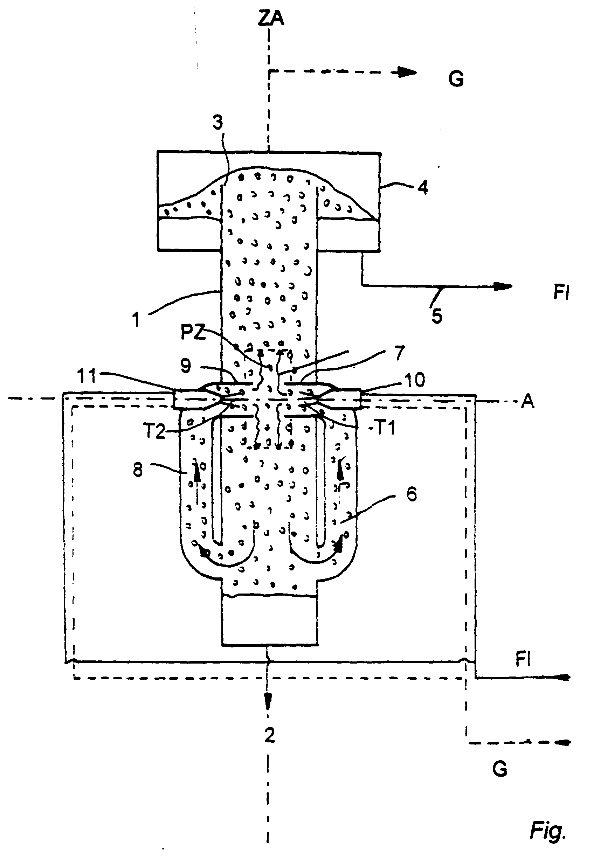

Nachfolgend wird die Erfindung anhand eines Ausführungsbeispiels näher erläutert. Die einzige Figur zeigt einen schematischen Querschnitt einer Vorrichtung zur Herstellung von Enzymen.

Ein zylindrischer Behälter 1 ist an einem ersten Ende 2 mit einem Flüssigkeitsablauf (hier nicht gezeigt) versehen. An einem zweiten Ende 3 ist ein Entgaser 4 mit einem weiteren Flüssigkeitsablauf 5 vorgesehen. Ein erster Bypaß 6 mündet mit einem ersten Rohrstück 7 und ein zweiter Bypaß 8 mündet mit einem zweiten Rohrstück 9 in den Behälter 1. Die durch das erste 7 und das zweite Rohrstück 9 gebildete Zuläufe liegen einander gegenüber. Im ersten Rohrstück 7 befindet sich eine erste 2-Stoffdüse 10 und im zweiten Rohrstück 9 befindet sich eine zweite 2-Stoffdüse 11. Die Öffnungen der ersten 10 und der zweiten 2-Stoffdüse 11 liegen einander gegenüber. Die erste 10 und die zweite 2-Stoffdüse 11 liegen auf einer gemeinsamen Achse A, welche senkrecht zur Behälterachse ZA steht. Die Pfeile deuten den Strömungsverlauf im Behälterinneren sowie im ersten 6 und zweiten Bypaß 8 an. Die erste 2-Stoffdüse 10 erzeugt einen ersten Triebstrahl T1 und die zweite 2-Stoffdüse 11 einen zweiten Triebstrahl T2. Mit PZ ist eine Prallzone bezeichnet, welche durch die aufeinandergerichteten Triebstrahlen T1 und T2 gebildet wird. Die Achsen der beiden Triebstrahlen T1 und T2 entsprechen der Achse A.

Die Funktion der Vorrichtung ist die folgende:

Zunächst wird der zylindrische Behälter 1 mit Flüssigkeit beschickt, welche mit Mikroorganismen versetzt ist. Am ersten Ende 2 wird Flüssigkeit entzogen und unter Druck der ersten 10 und der zweiten 2-Stoffdüse 11 als erster Stoff zugeführt.

Als zweiter Stoff wird den 2-Stoffdüsen 10 und 11 Luft zugeführt. Hinter den Öffnungen der 2-Stoffdüsen 9 und 11 bildet sich ein hochturbulentes Luft/Flüssigkeitsgemisch. Dieses trifft in der Prallzone PZ aufeinander. Durch die dort wirkenden Scherkräfte wird die Luft fein dispergiert. Es steht eine große volumenbezogene Stoffaustauschfläche zur Verfügung. Daher kann die Fermentation bis zu hohen Biomassegehalten fortgesetzt werden. Während der Umwälzung beträgt die Geschwindigkeit der Triebstrahlen T1 und T2 etwa 15 m/s. Die dabei erzeugten Scherkräfte reichen nicht aus, um die Mikroorganismen zu zerstören.

Um die Fermentation weiter zu unterstützen, wird dem Behälter Nährstofflösung am ersten Ende 2 zugeführt. Ein dem zugeführten Nährstofflösungsvolumen entsprechendes Volumen tritt am zweiten Ende 3 aus. Es gelangt in den Entgaser 4. Dem Entgaser 4 ist eine Separationsvorrichtung (hier nicht dargestellt) nachgeschaltet. Dort werden die Mikroorganismen aus der Flüssigkeit abgetrennt und in den Behälter 1 zurückgeführt. Die verbleibende mikroorganismenfreie Flüssigkeit wird über den Flüssigkeitsablauf 5 dem Verfahren entzogen.

Sobald eine vorgegebene Biomassekonzentration in der Flüssigkeit erreicht worden ist, wird die Zufuhr an Nährstofflösung und Luft abgestellt. Sodann wird die Triebstrahlgeschwindigkeit auf etwa 80 m/s erhöht. Durch die dann in der Prallzone PZ wirkenden hohen Scherkräfte werden die Mikroorganismen aufgeschlossen. Sobald der Aufschluß beendet ist, wird die Flüssigkeit aus dem zylindrischen Behälter 1 entnommen und die darin vorliegenden Enzyme werden abgetrennt.

### Bezugszeichenliste

- 1: zylindrischer Behälter
- 2: erstes Ende
- 3: zweites Ende
- 4: Entgaser
- 5: Flüssigkeitsablauf
- 6: erster Bypaß
- 7: erstes Rohrstück
- 8: zweiter Bypaß
- 9: zweites Rohrstück
- 10: erste 2-Stoffdüse
- 11: zweite 2-Stoffdüse
- T1: erster Triebstrahl
- T2: zweiter Triebstrahl
- PZ: Prallzone
- A: Düsenachsen
- ZA: Behälterachse

## Patentansprüche

1. Verfahren zur Herstellung von Enzymen aus in einer Flüssigkeit vorliegenden Mikroorganismen mit folgenden Schritten:
a) Umwälzung der Flüssigkeit durch zwei gegeneinander gerichtete eine erste Strömungsgeschwindigkeit aufweisende Triebstrahlen (T1, T2) und
b) Aufschluß der Mikroorganismen durch Erzeugen einer zweiten Strömungsgeschwindigkeit, die höher als die erste Strömungsgeschwindigkeit ist.

2. Verfahren nach Anspruch 1, wobei die erste Strömungsgeschwindigkeit höchstens 30 m/s, vorzugsweise 15 m/s, ist.

3. Verfahren nach Anspruch 1 oder 2, wobei die zweite Strömungsgeschwindigkeit zwischen 30 m/s und 100 m/s, vorzugsweise 80 m/s, ist.

4. Verfahren nach einem der vorhergehenden Ansprüche, wobei der Flüssigkeit während des Schritts lit.a eine Nährstofflösung für die Mikroorganismen und/oder Gas zugeführt wird.

5. Verfahren nach Anspruch 4, wobei die Nährstofflösung einen oder mehrere der folgenden Stoffe enthält: zellverwertbare Kohlenstoffverbindungen, insbesondere Acetate, Mineralien und Spurenelemente.

6. Verfahren nach einem der vorhergehenden Ansprüche, wobei während des Schritts lit.a Flüssigkeit entnommen wird.

7. Verfahren nach Anspruch 6, wobei die Mikroorganismen aus der entnommenen Flüssigkeit abgetrennt und zurückgeführt werden.

8. Verfahren nach einem der vorhergehenden Ansprüche, wobei der Schritt lit.b ohne Zufuhr von Nährstofflösung und/oder Gas durchgeführt wird.

9. Verfahren nach einem der vorhergehenden Ansprüche, wobei die Flüssigkeit beheizt wird.

10. Vorrichtung zur Herstellung von Enzymen aus in einer Flüssigkeit vorliegenden Mikroorganismen, wobei ein zylindrischer Behälter (1) mit mindestens zwei Bypaßrohren (6, 8) versehen ist, deren Zuläufe in einer gemeinsamen Behälterquerschnittsebene in den Behälter (1) münden, und wobei mindestens zwei Mittel (10,11) zur Erzeugung jeweils eines Triebstahls (T1, T2) vorgesehen sind, **dadurch gekennzeichnet,** daß die Mittel (10, 11) zur Erzeugung der Triebstrahlen (T1, T2) eine gemeinsame im wesentlichen senkrecht zur Behälterachse (ZA) stehende Achse (A) aufweisen, so daß die daraus austretenden Triebstrahlen (T1, T2) einander entgegengesetzt sind.

11. Vorrichtung nach Anspruch 10, wobei der Behälter (1) mit einer Einrichtung zum Zuführen von Gas verbunden ist.

12. Vorrichtung nach Anspruch 11, wobei die Zuläufe jeweils durch ein in das Behälterinnere ragendes Rohrstück (7, 9) gebildet sind.

13. Vorrichtung nach einem der Ansprüche 10 bis 12, wobei das Mittel (10, 11) zur Erzeugung des Triebstrahls mindestens eine Düse aufweist.

14. Vorrichtung nach Anspruch 13, wobei die Düse eine 2-Stoffdüse ist.

15. Vorrichtung nach einem der Ansprüche 13 oder 14, wobei in mindestens zwei einander gegenüberliegenden Zuläufen die jeweils mindestens eine Düse eines Mittels (10, 11) zur Erzeugung eines Triebstrahls (T1, T2) aufgenommen ist.

16. Vorrichtung nach einem der Ansprüche 10 bis 15, wobei am einen Ende (3) des zylindrischen Behälters (1) eine Einrichtung (4) zum Entgasen der Flüssigkeit vorgesehen ist.

17. Vorrichtung nach einem der Ansprüche 10 bis 16, wobei am einen Ende (3) eine Einrichtung zum Abtrennen der Mikroorganismen aus der Flüssigkeit und zum Zurückführen in den Behälter (1) vorgesehen ist.

18. Vorrichtung nach einem der Ansprüche 10 bis 17, wobei am anderen Ende (2) des zylindrischen Behälters (1) ein Flüssigkeitsablauf vorgesehen ist.
